# EUROPÄISCHE PATENTSCHRIFT

(11) **EP 2 788 493 B1**
(45) Veröffentlichungstag und Bekanntmachung des Hinweises auf die Patenterteilung: **15.05.2019**
(21) Anmeldenummer: 12788556.4
(22) Anmeldetag: 22.11.2012
(51) Int. Cl.: C12P 7/02, C12P 7/16, C12P 7/52, C12N 9/02

(54) **VERWENDUNG EINER ALKB ALKAN 1-MONOOXYGENASE ZUR OXIDATION VON PROPAN UND BUTAN**
USE OF AN ALKB ALKANE 1-MONOOXYGENASE FOR THE OXIDATION OF PROPANE AND BUTANE
UTILISATION D'UNE ALCANE 1-MONO-OXYGÉNASE ALKB POUR L'OXYDATION DU PROPANE ET DU BUTANE

(30) Priorität: 05.12.2011 EP 11191910
(43) Veröffentlichungstag der Anmeldung: 15.10.2014
(73) Patentinhaber: Evonik Degussa GmbH, 45128 Essen (DE)
(72) Erfinder: PFEFFER, Jan Christoph, 63452 Hanau (DE); HAAS, Thomas, 48161 Münster (DE); THUM, Oliver, 40880 Ratingen (DE); WITTMANN, Eva Maria, 83301 Traunreut (DE); GEHRING, Christian, 45770 Marl (DE); HAFKEMEYER, Sabine, 45770 Marl (DE); HÜLLER, Thomas, 45772 Marl (DE)
(74) Vertreter: Evonik Patent Association
(86) Internationale Anmeldenummer: PCT/EP2012/073334
(87) Internationale Veröffentlichungsnummer: WO 2013/083412

(56) Entgegenhaltungen:
- GRANT, C. ET AL.: "Whole-cell bio-oxidation of n-dodecane using the alkane hydroxylase system of P. putida GPo1 expressed in E. coli", ENZYME AND MICROBIAL TECHNOLOGY, Bd. 48, Nr. 6-7, 6. Mai 2011 (2011-05-06), Seiten 480-486, XP002667148, in der Anmeldung erwähnt
- JOHNSON, E.L. [PH D]: "Gasoline oxygenate biodegradation processes in Mycobacterium vaccae JOB5", , 2005, Seiten 110-140, XP008148917, ISBN: 0-542-14173-6 Gefunden im Internet: URL:http://search.proquest.com/docview/305 395658?accountid=29404
- VAN BEILEN, J.B. ET AL.: "Substrate specificity of the alkane hydroxylase system of Pseudomonas oleovorans GPo1", ENZYME AND MICROBIAL TECHNOLOGY, Bd. 16, Nr. 10, 1994, Seiten 904-911, XP023679643,
- FUJI, T. ET AL.: "Biotransformation of Various Alkanes Using the Escherichia coli Expressing an Alkane Hydroxylase System from Gordonia sp. TF6", BIOSCIENCE, BIOTECHNOLOGY, AND BIOCHEMISTRY, Bd. 68, Nr. 10, 2004, Seiten 2171-2177, XP002669751,
- MCKENNA, E.J. & COON, M.J.: "Enzymatic omega-Oxidation.IV. Purification and properties of the omega-hydroxylase of Pseudomonas oleovorans", JOURNAL OF BIOLOGICAL CHEMISTRY, Bd. 245, Nr. 15, 1970, Seiten 3882-3889, XP002671879,
- WUBBOLTS, M.G. ET AL.: "Biosynthesis of Synthons in Two-Liquid-Phase Media", BIOTECHNOLOGY AND BIOENGINEERING, Bd. 52, Nr. 2, 20. Oktober 1996 (1996-10-20), Seiten 301-308, XP000972414,
- GRANT, C. ET AL.: "Tools for Characterizing the Whole-Cell Bio-Oxidation of Alkanes at Microscale", BIOTECHNOLOGY AND BIOENGINEERING, Bd. 109, Nr. 9, 3. April 2012 (2012-04-03) , Seiten 2179-2189, XP002688926,

## Beschreibung

Die Erfindung betrifft die Verwendung einer Oxidoreduktase vom AlkB-Typ zur Herstellung eines Gemisches von Oxidationsprodukten eines Alkans, wobei das Verhältnis von Carbonsäure zu Alkohol unter den Oxidationsprodukten größer als 1:1 ist.

Alkane stellen einige der wichtigsten Grundstoffe der chemischen Industrie dar. Ausgangspunkt ihrer Gewinnung sind in der Regel fossile Rohstoffe, mittlerweile sind allerdings auch Verfahren zur Gewinnung von Alkanen aus nachwachsenden Rohstoffen bekannt. Während der Öffentlichkeit Alkane insbesondere aufgrund ihrer Verwendbarkeit als Energieträger, beispielsweise in Form von Gasen der kurzkettigen Alkane oder flüssigen längerkettigen Alkane bekannt sind, sind sie in der Industrie vor allem in ihrer Rolle als Edukte oder Lösungsmittel für zahlreiche Synthesen, die zu wichtigen Produkten des täglichen Lebens wie Kunststoffen oder Pharmazeutika führen, unentbehrlich.

Eine Grundbedingung für die Verwendung von Alkanen zu derartigen Zwecken ist die Möglichkeit der oxidativen Einführung von heteroatomhaltigen Funktionen in die Alkan-Kohlenstoffketten, z.B. von Hydroxy-, Keto- und Carboxyfunktionen, da Alkane *per se* aufgrund ihrer Sättigung chemisch als relativreaktionsträge zu charakterisieren sind. Dabei darf es, anders als bei der Verwendung von Alkanen als Heizmittel, jedoch nicht zu einer vollständigen Oxidation von Alkanen zu Kohlendioxid kommen, sondern die heteroatomhaltigen Funktionen müssen selektiv und kontrolliert eingeführt werden.

Zur synthetischen Darstellung von mit heteroatomhaltigen Funktionen substituierten Alkanen sind zahlreiche Reaktionen bekannt, beispielsweise die Halogenierung von Alkanen unter Einwirkung von UV-Licht, deren Produkte als Edukte für Synthese zahlreicher Verbindungen dienen können. So können Alkohole durch nukleophile Substitution des halogensubstituierten Alkans erhalten werden. Derartige Reaktionen verlangen jedoch häufig die Verwendung von giftigen und/oder umweltschädlichen Stoffe, beispielsweise von Chlorgas, das aufgrund seines niedrigen Preises industriell häufig zur Halogenierung eingesetzt wird.

Auch eine Reihe von biotechnologischen Verfahren zur Einführung von heteroatomhaltigen, insbesondere sauerstoffhaltigen, Funktionen in Alkane sind bekannt. So wird in einem Patent von Exxon (EP 98137) die Umsetzung von Propan zu Aceton durch *Arthrobacter petroleophagus* und andere Wildtypstämme beschrieben.

Grant et al. (2011) offenbaren die Verwendung einer Oxidoreduktase vom AlkB-Typ zur Herstellung in Anwesenheit von Sauerstoff eines Gemisches von Oxidationsprodukten eines Alkans,
wobei das Verhältnis von Carbonsäure zu Alkohol unter den Oxidationsprodukten größer als 1:1, nämlich 14:1,
wobei es sich bei dem Alkan um ein flüssiges Alkan mit 12 Kohlenstoffatomen handelt,
wobei es sich bei der Oxidoreduktase vom AlkB-Typ um AlkB aus *Pseudomonas putida* GPO1 handelt.

Johnson, E.L. ("Propane and n-Butane Oxidation by Pseudomonas putida GPo1" (2005), URL:http:// search.proquest.com/docview/305395658?accountid=29404) offenbart die Verwendung einer Oxidoreduktase vom AlkB-Typ zur Herstellung in Anwesenheit von Sauerstoff von Oxidationsprodukten eines Alkans,
wobei es sich bei dem Oxidationsprodukt um den Alkohol handelt,
wobei es sich bei dem Alkan um ein gasförmiges Alkan mit 3 oder 4 Kohlenstoffatomen handelt,
wobei es sich bei der Oxidoreduktase vom AlkB-Typ um AlkB aus *Pseudomonas putida* GPO1 handelt.

Vor diesem Hintergrund besteht die der Erfindung zugrunde liegende Aufgabe darin, ein biotechnologisches Verfahren zur Oxidation von Alkanen zu entwickeln, das zur selektiven Oxidation eines endständigen Kohlenstoffatoms eines Alkans bis hin zur Carbonsäure geeignet ist.

Weiterhin besteht die der Erfindung zugrunde liegende Aufgabe darin, ein Verfahren zu entwickeln, dass geeignet ist, um verschiedene selektiv am endständigen Kohlenstoffatom oxidierte Produkte des Alkans herzustellen, wobei die Menge bzw. das Verhältnis der Produkte beeinflusst werden kann.

Weiterhin besteht die der Erfindung zugrunde liegende Aufgabe darin, ein Oxidoreduktasesystem bereitzustellen, das die Oxidation eines endständigen Kohlenstoffatoms zu sämtlichen Oxidationsstufen aus der Gruppe umfassend Alkohol, Aldehyd und Carbonsäure zu katalysieren imstande ist, wobei nur ein einziges katalytisch aktives Polypeptid mit dem Substratalkan bzw. dessen Zwischenprodukten in Kontakt tritt.

Weiterhin besteht die der Erfindung zugrunde liegende Aufgabe darin, ein vom Fettsäurestoffwechsel unabhängiges und durch Überexpression eines einzelnen Oxidationssystems charakterisiertes System zur selektiven endständigen Oxidation von Alkanen bereitzustellen.

Eine weitere der Erfindung zu Grunde liegenden Aufgabe besteht darin, ein Verfahren zur Oxidation von Alkanen, bevorzugt von gasförmigen Alkanen, bereitzustellen, das geeignet ist, um das Alkan überwiegend bzw. mit verbesserter Ausbeute zur Carbonsäure, nicht lediglich überwiegend zum Alkohol zu oxidieren.

Diese und weitere Aufgaben werden durch den Gegenstand des beigefügten unabhängigen Anspruchs gelöst, wobei sich Ausführungsformen aus den Unteransprüchen ergeben.

In einem Aspekt wird das der Erfindung zu Grunde liegende Problem gelöst durch die Verwendung einer Oxidoreduktase vom AlkB-Typ zur Herstellung in Anwesenheit von Sauerstoff eines Gemisches von Oxidationsprodukten eines Alkans,
wobei das Verhältnis von Carbonsäure zu Alkohol unter den Oxidationsprodukten größer als 1:1 ist,
wobei es sich bei dem Alkan um ein gasförmiges Alkan mit 3 oder 4 Kohlenstoffatomen, bevorzugt um Butan, handelt, und
wobei es sich bei der Oxidoreduktase vom AlkB-Typ um AlkB aus *Pseudomonas putida* GPO1 oder eine Aminosäuresequenz, die mit Hinblick auf die Aminosäure-sequenz von AlkB aus *Pseudomonas putida* GPO1 eine Identität von 70 % oder mehr Prozent aufweist und die gleiche Funktion wie die ursprüngliche AlkB aufweist, handelt.

In einer ersten Ausführungsform des Aspektes handelt es sich bei dem Alkan um ein verzweigtes Alkan, bevorzugt mit vier Kohlenstoffatomen, noch bevorzugter Isobutan.

In einer zweiten Ausführungsform des Aspektes, bei der es sich auch um eine Ausführungsform der ersten Ausführungsform des Aspektes handelt, wird die Oxidoreduktase vom AlkB-Typ in Form von einem Ganzzellkatalysator bereitgestellt.

In einer dritten Ausführungsform des Aspektes, bei der es sich auch um eine Ausführungsform der ersten Ausführungsform des Aspektes handelt, wird die Oxidoreduktase vom AlkB-Typ in Form von einem aufgereinigten Polypeptid bereitgestellt.

In einer vierten Ausführungsform des Aspektes, bei der es sich auch um eine Ausführungsform der ersten bis dritten Ausführungsform des Aspektes handelt, ist das Verhältnis von Carbonsäure zu Alkohol unter den Oxidationsprodukten größer als 5:1, bevorzugt größer als 12:1 ist, noch bevorzugter größer als 20:1, am bevorzugtesten größer als 40:1 ist.

Die Erfinder der vorliegenden Erfindung haben festgestellt, dass Oxidoreduktasen vom AlkB-Typ, die in der Literatur als Katalysatoren zur Herstellung überwiegend von weniger stark oxidierten Produkten bekannt sind, überraschend verwendet werden können, um aus Alkanen, insbesondere aus gasförmigen Alkanen, überwiegend Produkte höherer Oxidationsstufen, insbesondere Carbonsäuren, ausgehend von Alkanen herzustellen. Insbesondere ist das Verhältnis von produzierten Carbonsäuren zu produzierten Alkoholen überraschend hoch. Weiterhin haben die Erfinder überraschend gefunden, dass derartige Oxidoreduktasen zur selektiven Oxidation von Alkanen befähigt sind, und zu erwartende Nebenprodukte, insbesondere an anderen als endständigen Kohlenstoffatomen oxidierte Alkane nur in unerwartet geringem Ausmaß oder überhaupt nicht in nachweisbaren Mengen herstellen.

Erfindungsgemäß werden gasförmige Alkanen mit 3 oder 4 Kohlenstoffatomen unter Verwendung einer Oxidoreduktase vom AlkB-Typ in Anwesenheit von Sauerstoff oxidiert. AlkB stellt eine zunächst aus dem AlkBGT-System aus *Pseudomonas putida* Gpo1 bekannt gewordene Oxidoreduktase dar, die von zwei weiteren Polypeptiden, AlkG und AlkT, abhängig ist. AlkT wird als FADabhängige Rubredoxin-Reduktase charakterisiert, die Elektronen aus NADH an AlkG weitergibt. Bei AlkG handelt es sich um ein Rubredoxin, ein eisenhaltiges Redoxprotein, das als direkter Elektronendonor für AlkB fungiert. Beispielsweise wird unter dem selben Begriff "Oxidoreduktase des alkB-Typs" ein Polypeptid mit einer Sequenzhomologie von z.B. wenigstens 75, 80, 85, 90, 92, 94, 96, 98 oder 99 % zur Sequenz des AlkB von *Pseudomonas putida* Gpo1 (Datenbankcode: CAB54050.1; dieser Datenbankcode stammt wie alle weiteren in der Anmeldung verwendeten aus dem Stand der Technik, nämlich aus der NCBI Datenbank, genauer dem am 15. November 2011 online verfügbaren Release) mit der Fähigkeit, Alkane zu oxidieren, verstanden. In einem weiteren Beispiel handelt es sich bei der Oxidoreduktase vom AlkB-Typ um eine mit den AlkG (CAB54052.1)- und AlkT (CAB54063.1)-Polypeptiden aus *Pseudomonas putida* Gpo1 funktionell zusammenwirkende, Alkane oxidierende Oxidoreduktase. Erfindungsgemäß handelt es sich bei der Oxidoreduktase vom AlkB-Typ um AlkB aus dem AlkBGT-System aus *Pseudomonas putida* Gpo1 oder eine Variante davon.

Die Lehre der vorliegenden Erfindung kann nicht nur unter Verwendung der exakten Aminosäure- oder Nukleinsäuresequenzen der hierin beschriebenen biologischen Makromoleküle ausgeführt werden, sondern auch unter Verwendung von Varianten derartiger Makromoleküle, die durch Deletion, Addition oder Substitution einer oder mehr als einer Aminosäuren oder Nukleinsäuren erhalten werden können. Erfindungsgemäß bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei z.B. andere als die das katalytisch aktive Zentrum ausbildende Aminosäuren oder für die Struktur oder Faltung essentielle Aminosäuren deletiert oder substituiert sind oder letztere lediglich konservativ substituiert sind, beispielsweise ein Glutamat statt einem Aspartat oder ein Leucin statt einem Valin. Es besteht keine Notwendigkeit, dass die Sequenz über ihre gesamte Länge eine entsprechend hohe Homologie aufweist, beispielsweise können auch Fusionsproteine oder für solche kodierende Nukleinsäuren verwendet werden, die einen Teil mit entsprechender Homologie und/oder Aktivität aufweist. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. Erfindungsgemäß weist die Variante einer Aminosäure- oder Nukleinsäuresequenz, zusätzlich zur oben genannten Sequenzhomologie, im Wesentlichen die gleiche enzymatisch Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. Beispielweise bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, z.B. oder eine Größenordnung von den K_{M}- und/oder k_{cat}- Werten unterscheidet, die das Wildtyppolypeptid mit Hinblick auf die gleichen Substrate aufweist. Beispielsweise umfasst der Begriff "Variante" einer Nukleinsäure- oder Aminosäuresequenz wenigstens einen aktiven Teil/oder Fragment der Nukleinsäure- bzw. Aminosäuresequenz. Beispielsweise bedeutet der Begriff
"aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Alkoholdehydrogenase, Monooxygenase oder Transaminase. In einem weiteren Beispiel umfasst der Begriff "Variante" einer Nukleinsäure eine Nukleinsäure, deren komplementärer Strang, z.B. unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in Ausubel *et al.* (1995) beschrieben. In einem anderen Beispiel umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Für viele Anwendungen wird die Oxidoreduktase vom AlkB-Typ verwendet als Teil eines Ganzzellkatalysators die Ausführungsform der Wahl sein, da sie keine oder zumindest vollständige Aufreinigung der Oxidoreduktase bzw. ihrer Aktivität erfordert. Beispielsweise wird unter dem Begriff "Ganzzellkatalysator", wie hierin verwendet, eine stoffwechselaktive Zelle verstanden, die eine enzymatische Aktivität von Interesse, z.B. die einer Oxidoreduktase vom AlkB-Typ, aufweist, z.B. in einem relativ zu ihrem Wildtyp erhöhten Maß, das vorteilhafterweise durch Überexpression einer rekombinanten Oxidoreduktase vom AlkB-Typ auf einem Plasmid oder integriert ins Genom erreicht werden kann. Dem Fachmann sind zahlreiche Systeme zur Herstellung von Ganzzellkatalysatoren bekannt, beispielsweise aus der DE 60216245. Beispielsweise handelt es sich bei der als Ganzzellkatalysator oder bei der als Expressionssystem verwendeten Zelle um eine prokaryotische, z.B. um eine bakterielle Zelle. In einem weiteren Beispiel handelt es sich um eine Säugetierzelle. In einem anderen Beispiel handelt es sich um eine niedere eukaryotische Zelle, z.B. um eine Hefezelle. Beispielhafte prokaryotische Zellen umfassen *Escherichia,* besonders *Escherichia coli,* und Stämme der Gattung *Pseudomonas* und *Corynebacterium.* Beispielhafte niedere eukaryotische Zellen umfassen die Gattungen *Saccharomyces, Candida, Pichia, Yarrowia, Schizosaccharomyces,* besonders die Stämme *Candida tropicalis, Schizosaccharomyces pombe, Pichia pastoris, Yarrowia lipolytica* und *Saccharomyces cerivisiae.* Die Zelle kann eine oder mehr als eine für ein erfindungsgemäß verwendetes Enzym kodierende Nukleinsäuresequenz auf einem Plasmid oder integriert in ihrem Genom enthalten. Verfahren zur Herstellung derartiger Plasmide bzw. Zellen kann der Fachmann routinemäßig ausführen. Sie sind in Lehrbüchern und Versuchsprotokollsammlungen der Molekularbiologie, Biochemie, Genetik und Mikrobiologie beschrieben, beispielsweise in Sambrook *et al.* (1989).

In einer weiteren bevorzugten Ausführungsform, handelt es sich bei der Oxidoreduktase vom AlkB-Typ jedoch um ein aufgereinigtes Enzym. Dabei kann es sich, wie bei allen erfindungsgemäß eingesetzten enzymatisch aktiven Polypeptiden, um Lysate von Zellen oder Präparationen der Polypeptide in sämtlichen Aufreinigungsstufen, vom kruden Lysat bis zum reinen Polypeptid, handeln. Beispielsweise ist unter dem Begriff "aufgereinigtes" Enzym, wie hierein verwendet, zu verstehen, dass nicht die ganze Zelle oder ein unverarbeitetes Extrakt von ihr zur Katalyse eingesetzt wird, sondern das Enzym teilweise oder vollständig aufgereinigt wird. In einem weiteren Beispiel bedeutet der Begriff "aufgereinigtes" Enzym, wie hierin verwendet, dass das Enzyme soweit aufgereinigt ist, dass es auf einem SDS-Gel der Präparation wenigstens ca. 80, 85, 95, 98 oder 99 % der sichtbaren Proteine darstellt. In einem anderen Beispiel ist das Enzym soweit aufgereinigt, dass es das einzige erkennbare Polypeptid auf einem SDS-Gel der entsprechenden Präparation darstellt. Dem Fachmann auf dem Gebiet sind zahlreiche Verfahren bekannt, mit denen enzymatisch aktive Polypeptide in geeigneten Zellen überexprimiert und aufgereinigt bzw. isoliert werden können. So können zur Expression der Polypeptide sämtliche dem Fachmann zur Verfügung stehende Expressionssysteme verwendet werden, beispielsweise Vektoren vom Typ pET oder pGEX. Zur Aufreinigung kommen chromatographische Verfahren in Frage, beispielsweise die affinitätschromatographische Aufreinigung eines mit einem Tag versehenen rekombinanten Proteins unter Verwendungen eines immobilisierten Liganden, beispielsweise eines Nickelions im Falle eines Histidin-Tags, von immobilisiertem Glutathion im Falle einer an das Zielprotein fusionierten Glutathion-S-Transferase oder von immobilisierter Maltose im Falle eines Tags umfassend Maltose-bindendes Protein.

Die aufgereinigten enzymatisch aktiven Polypeptide können entweder in löslicher Form oder immobilisiert eingesetzt werden. Dem Fachmann sind geeignete Verfahren bekannt, mit denen Polypeptide an organischen oder anorganischen Festphasen kovalent oder nichtkovalent immobilisiert werden können, beispielsweise durch Sulfhydryl-Kupplungs-Chemie (z.B. Kits der Firma Pierce).

Die erfindungsgemäße Lehre wird auf gasförmige Alkane mit 3 oder 4 Kohlenstoffatomen angewendet.

Zur Durchführung des Verfahrens kommen verschiedene Bedingungen in Frage. Essentiell ist die Anwesenheit von molekularem Sauerstoff als Oxidationsmittel. Der Sauerstoff kann in Form von Sauerstoffquellen wie Wasserstoffperoxid oder Kaliumpermanganat vorliegen und aus diesen *in situ* gebildet werden, ein weiteres Beispiel ist jedoch die Einleitung von Sauerstoffgas, z.B. in Form von Luft, in das die Oxidoreduktase umfassende flüssige Reaktionsmedium. Die Temperatur kann dabei mit der Maßgabe, dass im Falle der Verwendung einer lebenden Zelle bzw. einer Präparation geeigneter Enzyme die gewählte Zelle bzw. die gewählten Enzyme lebensfähig sind bzw. Aktivität zeigen, mehr als 20 °C, 30 °C, 40 °C, 50 °C, 60 °C, 70 °C oder mehr als 80 °C, z.B. bis 100 °C betragen. Dem Fachmann ist bekannt, welche Organismen bei welchen Temperaturen lebensfähig sind, beispielsweise aus Lehrbüchern wie Fuchs/Schlegel, 2007. Im Falle einer lebenden Hefezelle kann die Temperatur 5 bis 45 °C, z.B. 15 bis 42 °C, oder 20 bis 30 °C betragen. Im Falle eines Gram-negativen Bakteriums, z.B. eines Bakteriums aus der Familie der *Enterobacteriaceae,* wie *E. coli,* kann die Temperatur 5 bis 45 °C, z.B. 15 bis 42 °C, oder 20 bis 30 °C, oder 35 bis 40 °C betragen. Der pH-Wert muss derart sein, dass die Aktivität der Oxidoreduktase vom AlkB-Typ zumindest ausreichend lange erhalten bleibt. Im Falle der Verwendung eines Ganzzellkatalysators muss die Zelle ausreichend lange intakt bleiben. Beispielsweise kann der pH-Wert zwischen 3 und 12, z.B. 5 und 9, oder 6 und 8 liegen.

Das Kontaktieren des Alkans mit der Oxidoreduktase vom AlkB-Typ erfolgt in der Weise, dass die aufgereinigte oder in Form von einem Ganzzellkatalysator vorliegende Oxidoreduktase vom AlkB-Typ in wässriger Lösung in ausreichend stabiler Form vorliegt und das Alkan unter sanftem Rühren zusammen mit Sauerstoff zur Lösung hinzugegeben wird, falls es sich um ein festes oder flüssiges Alkan handelt, oder in Form eines Gases, sofern es sich um ein gasförmiges Alkan handelt, in die wässrige Lösung eingeleitet wird. Der Fachmann ist in der Lage, im Rahmen routinemäßiger Experimente stabilisierte Formen des Enzyms oder Ganzzellkatalysators bereitzustellen. Zu beachtende Faktoren wie der Einsatz eines geeigneten Puffersystems, die Einstellung geeigneter Temperatur-, pH- und Salzkonzentrationswerte sind in der Literatur beschrieben, beispielsweise in Cornish-Bowden, 1995.

Zur Anzucht der Zelle kommen zahlreiche Kulturmedien in Frage, im Falle der Verwendung einer Hefezelle beispielsweise YPD, YPN und YNB, die mit Aminosäuren, beispielsweise mit 0,01 g/L Tryptophan, oder mit Glucose, beispielsweise in einer Konzentration von 1 % (w/v), supplementiert sein können. Im Falle der Verwendung eines Bakteriums aus der Familie der *Enterobacteriaceae,* wie *E. coli,* kommen zur Anzucht Vollmedien wie LB-Medium oder Hochzelldichtemedium (HZD-Medium) bestehend aus NH₄SO₄ 1,76 g, K₂HPO₄ 19,08 g, KH₂PO₄ 12,5 g, Hefeextrakt 6,66 g, Na₃-Citrat 1,96 g , NH₄Fe-Citrat (1 %) 17 ml, Spurenelementelösung US3 5 ml, Feedlösung (Glucose 50 % w/v, MgSO₄ [x 7 H₂O 0,5 % w/v, NH₄Cl 2,2 % w/v) 30 ml pro Liter in Frage.

Beispielswweise werden im Verfahren verwendete Zellen in einem anderen Medium als demjenigen herangezogen, das für die Alkanoxidation verwendet wird. Beispielsweise handelt es sich bei dem für die Anzucht verwendeten Medium um ein Vollmedium und um das für Alkanoxidation verwendete Medium um ein Minimalmedium. Das Verfahren, sofern es unter Verwendung lebensfähiger Zellen durchgeführt wird, wird nach Anzucht der Zellen z.B. in Transformationspuffer durchgeführt, der pro Liter (NH₄)H₂PO₄ 8 g, NaCl 0,5 g, MgSO₄x 7 H₂O 0,48 g, Spurenelementlösung US3 15 ml. 1 Liter der Spurenelementelösung US3 setzte sich zusammen aus HCl 37 % 36,5 g, MnCl₂ x 4H₂O 1,91 g, ZnSO₄ x 7H₂O 1,87 g, Na-EDTA x 2H₂O 0,8 g, H₃BO₃ 0,3 g, Na₂MoO₄ x 2H₂O 0,25 g, CaCl₂ x 2H₂O 4,7 g, FeSO₄ x 7 H₂O 17,8 g, CuCl₂ x 2H₂O 0,15 g umfasst und dessen pH auf 5,4 eingestellt wird. Beispielsweise wird die Alkanoxidation in M9-Medium durchgeführt (15 g Glucose, 6,79 g Na₂PO₄, 3 g KH₂PO₄, 0,5 g NaCl, 2 g NH₄Cl, 15 g Hefeextrakt, 0,49 g MgSO₄*7H₂O, 1 ml TE und 50 µg Kanamycin in 1000 ml Schüttelkolben überimpft, wobei die Spurenelementlösung (TE) pro Liter wie folgt angesetzt wird: 36,5 g HCl 37 %, 1,91 g MnCl₂*4H₂O, 1,87 g ZnSO₄*7H₂O, 0,84 g Na-EDTA*2H₂O, 0,3 g H₃BO₃, 0,25 g Na₂MoO₄*2H₂O, 4,7 g CaCl₂*2H₂O, 17,3 g FeSO₄*7H₂O und 0,15 g CuCl₂*2H₂O).

Das Verfahren kann bei Atmosphärendruck durchgeführt. Im Falle der Verwendung gasförmiger Alkanedukte kann es jedoch vorteilhaft sein, die Oxidoreduktase vom AlkB-Typ die Reaktion bei höheren Drücke in Anwesenheit eines Gasgemisches katalysieren zu lassen, das überwiegend eine Mischung aus Alkanen und Sauerstoff umfasst, z.B. mehr als 50, 60, 70 oder 80 Volumenprozent. Beispielsweise beträgt der Druck mehr als 1,5, 2, 3 oder 4 bar. In einem weiteren Beispeil beträgt der Druck 0,5 bis 4, z.B. 1 bis 3, oder 1 bis 1,5 bar.

Ein besonderer Vorteil des Verfahrens besteht darin, dass bestimmte Verhältnisse der Oxidationsprodukte des als Edukt eingesetzten Alkans erhalten werden können. Das Alkan kann von der Oxidoreduktase vom AlkB-Typ an einem endständigen Kohlenstoffatom, d.h. einem Kohlenstoffatom, das kovalent nur mit höchstens einem weiteren Kohlenstoffatom direkt verbunden ist, prinzipiell zu drei Oxidationsstufen oxidiert werden, nämlich dem Alkohol, dem Aldehyd und der Carbonsäure. Erfindungsgemäß bedeutet die Angabe, dass "das Verhältnis von Carbonsäure zu Alkohol unter den Oxidationsprodukten bevorzugt größer als 1:1 ist", dass das Stoffmengenverhältnis von Carbonsäure zu Alkohol, also das Stoffmengenverhältnis von durch Oxidation eines endständigen Kohlenstoffatoms entstandener Carbonsäure zu durch Oxidation eines endständigen Kohlenstoffatoms entstandenem Alkohol größer als 1:1 ist, d.h. im Produktgemisch sind mehr Moleküle der durch Oxidation eines endständigen Kohlenstoffatoms entstandenen Carbonsäure als Moleküle des durch Oxidation eines endständigen Kohlenstoffatoms entstandenen Alkohols vorhanden.

In einer bevorzugten Ausführungsform kann die AlkB von *Pseudomonas putida* Gpo1, verwendet werden, um aus einem gasförmigen Alkan mit 3 oder 4 Kohlenstoffatomen in Anwesenheit von Sauerstoff Oxidationsprodukte herzustellen, wobei das Verhältnis von Carbonsäure zu Alkohol bevorzugt größer als 1:1, zunehmend bevorzugter 1,5:1, 2:1, 5:1, 10:1, 15:1 oder 20:1.

Offenbart sind ein Verfahren zur Oxidation eines endständigen Kohlenstoffatoms eines nichtzyklischen Alkans zu einem entsprechenden Aldehyd und/oder einer entsprechenden endständigen Monocarbonsäure, umfassend Kontaktieren des Alkans mit einem biologischen Agens, das eine katalytisch aktive Oxidoreduktase umfasst, in Anwesenheit von einem Oxidationsmittel, wobei es sich bei dem Alkan um Butan oder Isobutan handelt und wobei es sich bei der Oxidoreduktase um eine Oxidoreduktase vom alkB-Typ, z.B. um die Monooxygenase AlkB aus *P. putida* GPO1 oder ein Homologon davon handelt und das Oxidationsmittel Sauerstoff ist und eine Verwendung einer Oxidoreduktase vom alkB-Typ, z.B. die Monooxygenase AlkB aus *P. putida* GPO1 oder einem Homologon davon zur Oxidation eines endständigen Kohlenstoffatoms eines nichtzyklischen Alkans zu einem entsprechenden Aldehyd und/oder einer entsprechenden endständigen Monocarbonsäure, wobei es sich bei dem Alkan um Butan oder Isobutan handelt.

Die vorliegende Erfindung wird weiterhin durch die folgenden Figuren und Beispiele veranschaulicht.
**Figs. 1a****), b), c)** und **d)** zeigen die Konzentration von 1-Butanol (**a)**), 2-Butanol (**b)**), Butyraldehyd (**c)**) bzw. Buttersäure (**d)**) im Zeitverlauf bei Umsetzung von Butan mit Sauerstoff durch das Monooxygenase-System alkBGT aus *P. putida* GPO1 bei einer Rührerdrehzahl von 500 - 800 oder 900 Upm. Es werden die Konzentrationen im Fermenter (F) und in der Waschflasche (WF) gezeigt.
**Figs. 2 a)** und **b)** zeigen den Einfluss der Biomassekonzentration auf die Oxidation von Butan durch *E*. *coli* durch das Monooxygenase-System alkBGT aus *P. putida* GPO1, genauer die Konzentration von 1-Butanol (**a)**) und Buttersäure (**b)**) im Zeitverlauf.
**Figs. 3a****), b), c)** und **d)** zeigen den Einfluss der Konzentration an Spurenelement (TE)-Lösung auf die Oxidation von Butan durch *E*. *coli* durch das Monooxygenase-System alkBGT aus *P*. *putida* GPO1, genauer die Konzentration von 1-Butanol (**a)**), 2-Butanol (**b)**), Butyraldehyd (**c)**) bzw. Buttersäure (**d)**) im Zeitverlauf
**Figs. 4a****), b), c)** und **d)** zeigen einen Stämmevergleich *E*. *coli* BL21 mit *E*. *coli* W3110 durch das Monooxygenase-System alkBGT aus *P. putida* GPO1 und deren Einfluss auf die Oxidation von Butan durch *E*. *coli* mit der Monooxygenase (alkBGT) aus *P. putida* GPO1, genauer die Konzentration von 1-Butanol (**a)**), 2-Butanol (**b)**), Butyraldehyd (**c)**) bzw. Buttersäure (**d)**) im Zeitverlauf
**Figs. 5a****), b), c)** zeigen die Oxidation von iso-Butan durch *E*. *coli* durch das Monooxygenase-System alkBGT aus *P. putida* GPO1, genauer die Konzentration von 2-Methyl-1-propanol (**a)**), iso-Butyraldehyd (**b)**) und iso-Buttersäure (**c)**) im Zeitverlauf.
**Fig. 6** zeigt den für Beispiel 7 klonierten Vektor p-LL-30 schematisch.
**Fig. 7** zeigt die Oxidation von Butan durch *E.coli* mit dem Monooxygenase-System alkBG aus *Alcanivorax borkumensis,* wie in Beispiel 7 durchgeführt.

### Beispiel 1: Oxidation von Butan durch E. coli durch das Monooxygenase-System alkBGT aus P. putida GPO1

100µl einer Glycerin-Kryokultur von *E. coli* BL21 pCOM10 (Leerplasmid) und *E. coli* BL21 pBT10 (WO 2009/077461) werden auf einer LB-Agarplatte mit 50 µl Kanamycin ausplattiert und für 24 h bei 37°C inkubiert. Die LB-Platten werden aus 1 Liter einer Lösung von Hefeextrakt 5 g, Pepton 10 g, NaCl 0,5 g, Agar-Agar 15 g und Kanamycin 50 µg hergestellt. Der pH wird vor dem Autoklavierten mit 5 % NH₄OH auf 7,4 eingestellt.

Von diesen Platten werden (für einen Konversionsansatz) 2 x 25 ml LB-Broth (obige Lösung ohne Agar-Agar) mit 50 µl Kanamycin in einem 100 ml-Schüttelkolben mit Schikanen mit einem vollen Loop einer Impföse (Kapazität 10 µl) angeimpft. Die Kulturen werden für 24 h bei 37 °C und 200 Upm (Amplitude 2,5 cm) inkubiert.

Danach werden je 25 ml der Kulturbrühe in 75 ml modifiziertes M9-Medium (steril filtriert) mit folgender Zusammensetzung pro Liter: 15 g Glucose, 6,79 g Na₂PO₄, 3 g KH₂PO₄, 0,5 g NaCl, 2 g NH₄Cl, 15 g Hefeextrakt, 0,49 g MgSO₄*7H₂O, 1 ml TE und 50 µg Kanamycin in 1000 ml Schüttelkolben überimpft. Die Spurenelementlösung (TE) wird pro Liter wie folgt angesetzt: 36,5 g HCl 37 %, 1,91 g MnCl₂*4H₂O, 1,87 g ZnSO₄*7H₂O, 0,84 g Na-EDTA*2H₂O, 0,3 g H₃BO₃, 0,25 g Na₂MoO₄*2H₂O, 4,7 g CaCl₂*2H₂O, 17,3 g FeSO₄*7H₂O und 0,15 g CuCl₂*2H₂O. Der pH wird mit 5 % NH₄OH auf 7,4 eingestellt. Des Weiteren werden pro Kolben 3 Tropfen autokaviertem Antischaum (Delamex) und zugegeben.

Die Kolben werden für 2 Stunden bei 37°C und 180 Upm (Amplitude 2,5 cm) inkubiert. Danach wird die Temperatur auf 25 °C reduziert. Nach 0,5 Stunden bei 25 °C erfolgt die Induktion mit 0,4 mM DCPK. Die Kultur wird für weitere 16 Stunden bei 25 °C und 180 Upm geschüttelt. Danach erfolgt eine mikroskopische Prüfung auf Monosepsis.

Die Kulturen werden vereinigt, in 50 ml-Falcon Tubes abgefüllt und bei 10000 g bei 25 °C für 10 Minuten zentrifugiert. Der Überstand wird verworfen. Die Pellets aus 200 ml Kultur werden in 10 ml Konversionspuffer resuspendiert. Der Konversionspuffer besteht aus 70 mM Na⁺/K⁺ phosphatpuffer, pH 7, eingestellt mit 1 M NaOH, mit 6,79 g Na₂PO₄, 3 g KH₂PO₄, 0,5 g NaCl, 0,49 g MgSO₄*7H₂O, 1 ml TE und 50 µg Kanamycin oder aus 70 mM (NH₄)H₂PO₄ Puffer, pH 7 mit 8 g (NH₄)H₂PO₄, 0,5 g NaCl, 0,49 g MgSO₄*7H2O, 1 ml TE und 50 µg Kanamycin pro Liter. Die pH-Einstellung erfolgt hier mit 5 % NH₄OH.

170 ml Puffer mit ca. 3 Tropfen autokaviertem Antischaum (Delamex) werden in einem 300 ml-Fermenter vorgelegt. Der Fermenter wird mit einem Gasgemisch aus einer Gasflasche mit 5 bar Anfangsdruck von 25 % Butan und 75 % synthetischer Luft über einen Sinterglasperlator mit einer Porengröße von 0,2 µm mit einer Fußrate von 25 l/h begast. Der Fermenter wird in einem Wasserbad auf 25 °C temperiert und mit Hilfe eines Magnetrührers mit 500 Upm für 2 Stunden, danach mit 800 Upm gerührt. Die Abluft wird durch eine Waschflasche, die mit 150 ml Wasser gefüllt ist, abgeleitet.

Der Fermenter wird mit 10 ml des resuspendierten Pellets angeimpft. Die OD beider Kulturen liegt bei ca. 10. Die Reaktion wird durch Zugabe von 1 Vol.-% Glucose gestartet. Der pH kann wahlweise über den Versuchszeitraum ungeregelt oder geregelt sein. Nach 10, 45, 135 und 240 Minuten werden aus dem Fermenter und der Waschflasche jeweils 10 ml Probe gezogen. In den Proben aus dem Fermenter wird mit 2 ml HCl die Reaktion gestoppt. Die Fermenterproben werden 10 Minuten bei 10000 g und Raumtemperatur zentrifugiert und der Überstand über einen 0,2 µm-Spritzenvorsatzfilter filtriert. Die Proben werden für die Analytik in HPLC Vials abgefüllt. Die Analytik erfolgte chromatographisch mit HPLC-RID an einer Agilent Technologies 1200-Anlage. Es wurde eine Aminex HPX-87H-Säule (300mmx 7,8mm) verwendet. Die Anlage wurde mit 10 mM H₂SO₄ als Laufmittel bei einer Flussrate von 0,6 ml/min und einer Säulentemperatur von 40°C betrieben. Standards aller zu analysierender Stoffe wurden in Reinstwasser vorbereitet und unter identischen Bedingungen gemessen. Die Auswertung erfolgte über Vergleich der Retentionszeiten. Zusätzlich wird zu jedem Probenahmezeitpunkt eine 2 ml-Probe zu Bestimmung von pH, OD und Glucosekonzentration aus dem Fermenter gezogen. Der pH wird an einem externen pH-Meter gemessen, die OD spektrometrisch bei 600 nm und der Glucosegehalt mit einem biochemischen Analysator (YSI Select 2700 Von Kreienbaum) bestimmt.

### Ergebnisse

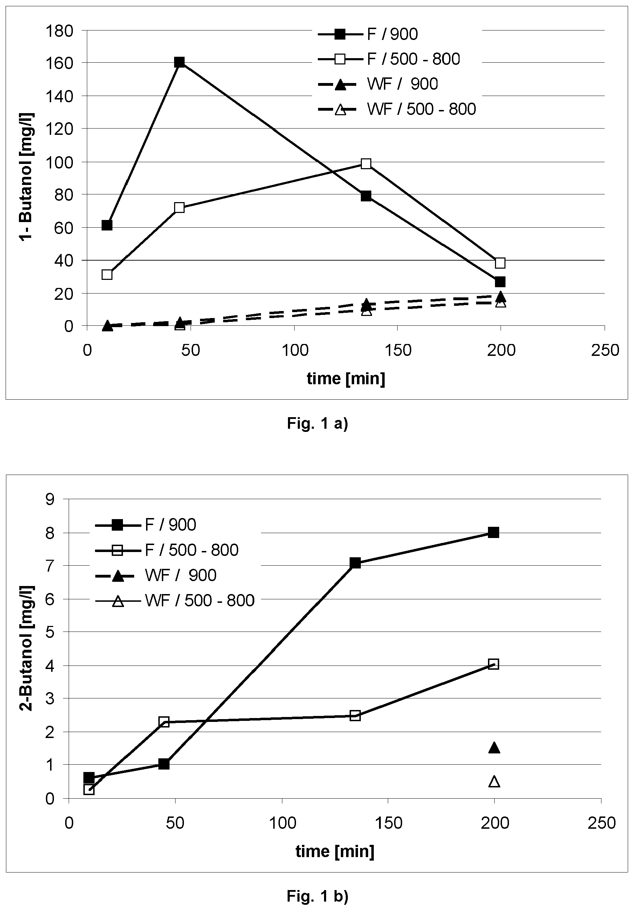

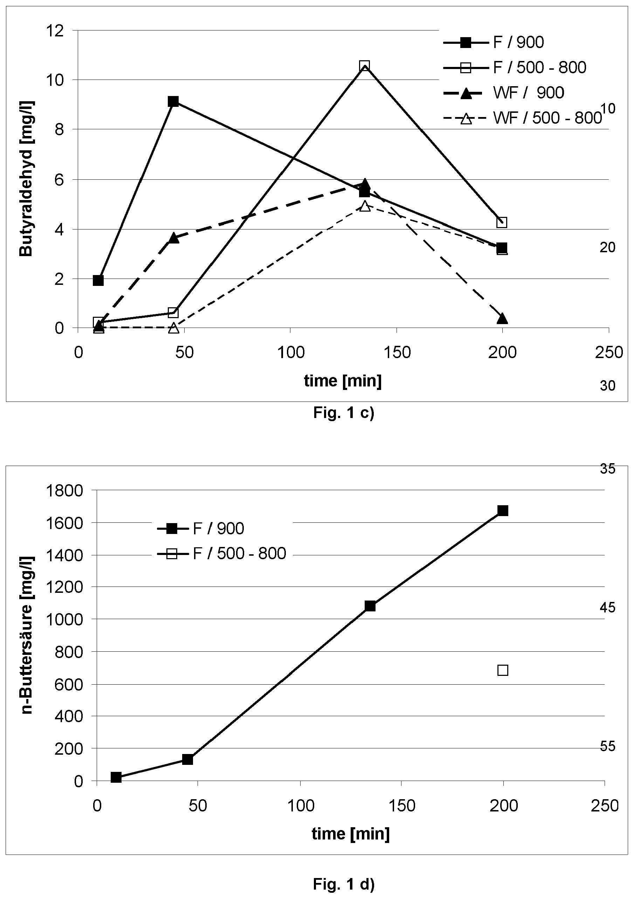

Die Ergebnisse sind in den **Fig. 1 a)** **- d)** dargestellt. Bei den Versuchen mit *E. coli* BL21 pCOM10 (Leerplasmid) findet keine Oxidation von Butan bzw. 1-Butanol statt. Hingegen werden mehr Verwendungen von *E. coli* BL21pBT10 als Oxidationsprodukten von n-Butan: 1-Butanol, Buttersäure, 2-Butanol, Butyraldehyd, 1,4-Butandiol (nicht quantifizierbar) und Butyrolacton (in Spuren) gefunden.

Die Konzentration aller Oxidationsprodukte steigt über den gesamten Versuchszeitraum an. Es wird ca. 1 g/lh Glucose verbraucht, der pH-Wert sinkt von 7 auf ca 5.

### Beispiel 2: Einfluss der Rührerdrehzahl (Stofftransportlimitierung) auf die Oxidation von n-Butan durch E. coli mit der Monooxygenase (alkBGT) aus P. putida GPO1

Der Versuch wird analog dem Beispiel 1 durchgeführt. Die Rührerdrehzahl wird in einem 2. Ansatz von Beginn an konstant auf 900 Upm gestellt. Die OD ist im Vergleich zu Beispiel 1 doppelt so hoch. Die TE-Konzentration ist 15fach. Die letzte Probenahme erfolgte nach 200 Minuten.

### Ergebnisse

Die Bildung von 1-Butanol im Fermenter (F) erfolgt bei ständig höherer Drehzahl schneller und erreicht eher ihr Maximum. In den Waschflaschen (WF) ist die Konzentration von 1-Butanol auf niedrigem Niveau annähernd identisch. Die Konzentration von 2-Butanol im Fermenter (F) steigt bei jeder Rührerdrehzahl über den gesamten Versuchszeitraum an, bleibt aber gering. In den Waschflaschen ist 2-Butanol erst am Ende der Versuchszeit nachweisbar. Die Konzentration von Butyraldehyd steigt mit höherer Rührerdrehzahl schneller, wird aber da der Dampfdruck bei 113 hPa (20°C) liegt auch schneller ausgetrieben. Butyraldehyd ist so nur qualitativ, nicht aber quantitativ nachweisbar.

n-Buttersäure wird bei niederer Rührerdrehzahl erst am Ende des Versuchszeitraums gebildet. Mit hoher Rührerdrehzahl steigt die Konzentration kontinuierlich an. In den Waschflaschen kann keine n-Buttersäure nachgewiesen werden.

### Beispiel 3: Einfluss der Biomassekonzentration auf die Oxidation von n-Butan durch E. coli mit der Monooxygenase (alkBGT) aus P. putida GPO1

Der Versuch wird analog dem Beispiel 1 durchgeführt. Die Rührerdrehzahl liegt konstant bei 900 Upm, die TE Konzentration ist jeweils 15fach. 1x bedeutet eine OD von ca. 10, 2x entsprechend 20.

### Ergebnisse

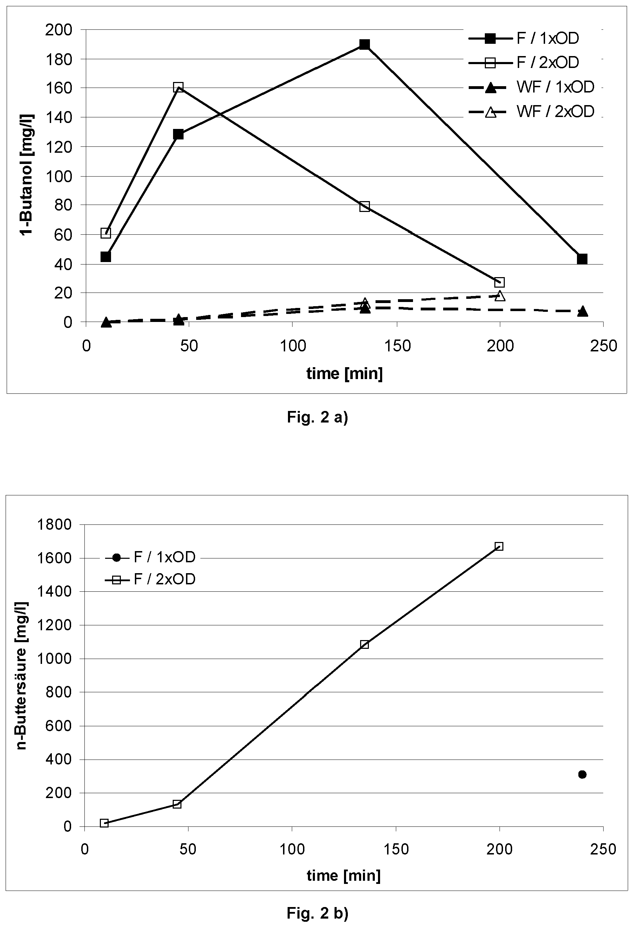

Die Ergebnisse sind in den **Fig. 2 a)** und **b)** dargestellt. Die Maximalkonzentration wird mit doppelter OD zu einem früheren Versuchzeitpunkterreicht erreicht. 1-Butanol wird dann auch schneller umgesetzt.

Buttersäure kann nur im Fermenter (F), nicht in den Waschflaschen nachgewiesen werden. Mit der doppelten OD beginnt die Buttersäurebildung bereits zu Beginn der Konversion. Mit einfacher OD kann unter diesen Bedingungen erst nach 240 Minuten Buttersäure nachgewiesen werden. Die Konzentration beträgt etwa 18% der Maximalkonzentration bei doppelter OD.

### Beispiel 4: Einfluss der TE-Konzentration auf die Oxidation von n-Butan durch E. coli mit der Monooxygenase (alkBGT) aus P. putida GPO1

Der Versuch wird analog dem Beispiel 1 durchgeführt. Die Rührerdrehzahl beträgt konstant 900 Upm. Der verwendete Stamm ist *E*. *coli* W3110 pBT10. Die Konzentration an TE ist 1 ml/l Puffer (1x) bzw. 15 ml/l Puffer (15x). Im Versuch mit der 15fachen Konzentration sind zusätzlich mit 30 mg/l MOPS zugegeben.

### Ergebnisse

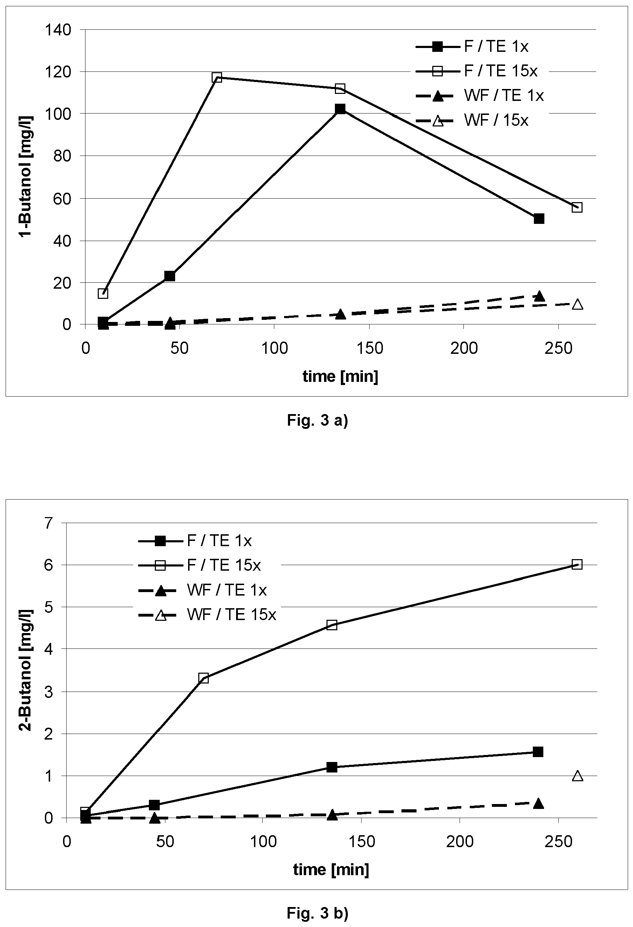

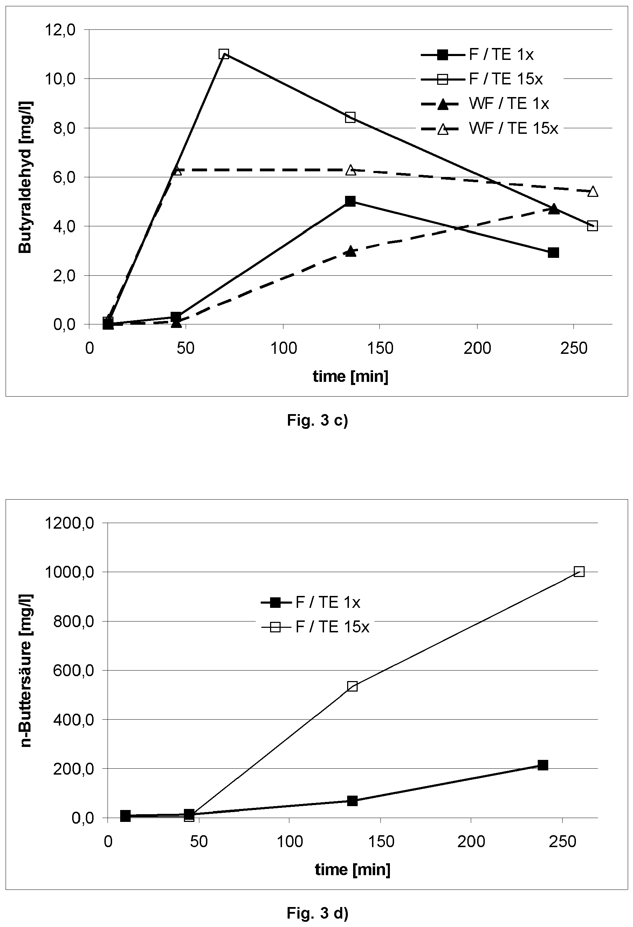

Die Ergebnisse werden sind in den **Figs. 3 a)** **- d)** dargestellt. Bei der 15 fachen TE Konzentrationen werden alle Oxidationsprodukte schneller und in höherer Konzentration gebildet.

### Beispiel 5: Stämmevergleich E. coli BL21 mit E. coli W3110 mit der Monooxygenase (alkBGT) aus P. putida GPO1

Der Versuch wird analog dem Beispiel 1 mit fester Rührerdrehzahl 900 Upm durchgeführt. Die TE-Konzentration beträgt 15 ml/l Konversionspuffer.

### Ergebnisse

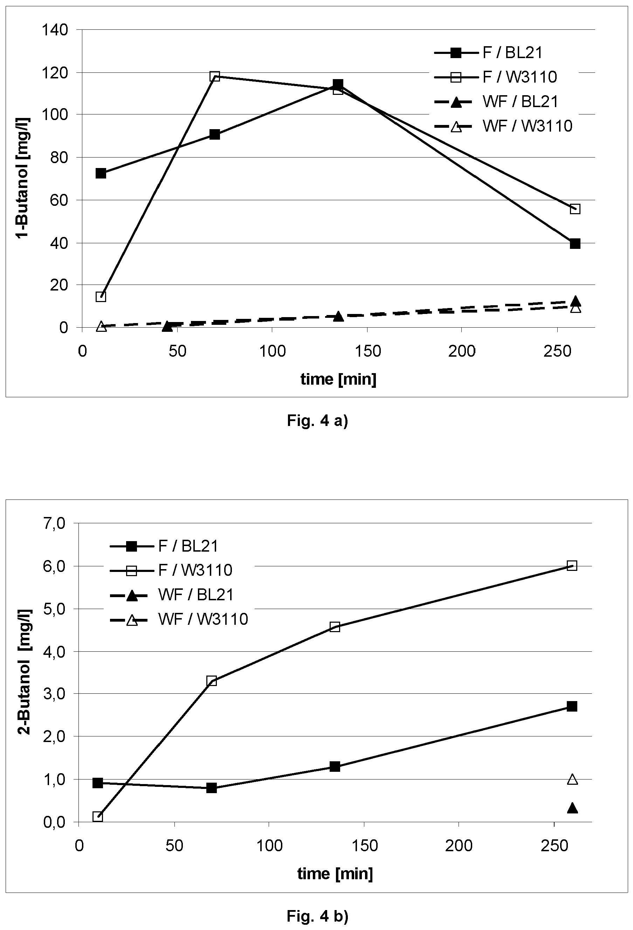

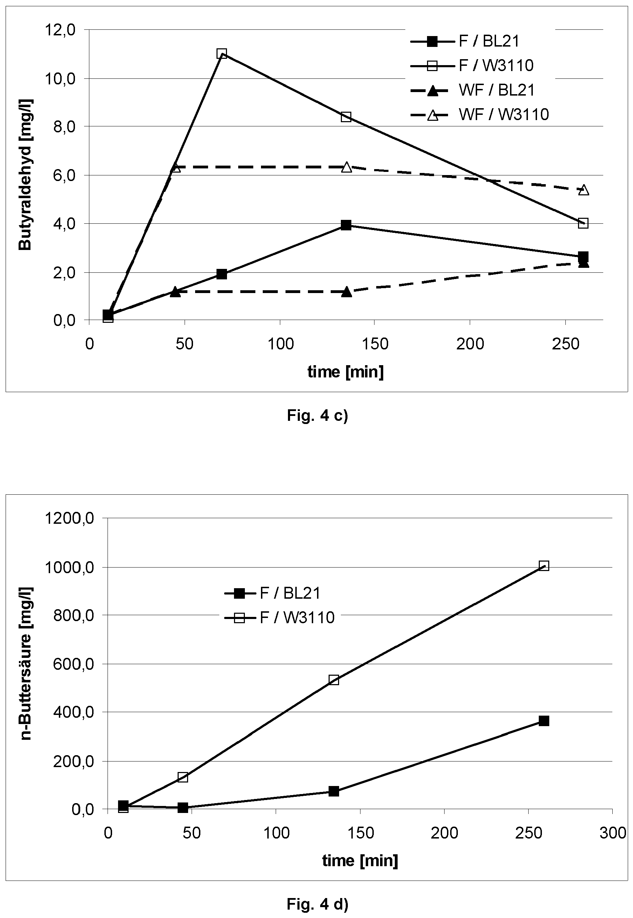

Die Ergebnisse sind in den **Figs. 4 a)** **- d)** dargestellt. Der Stamm *E. coli* W3110 pBT10 bildet alle Oxidationsprodukte schneller und in höheren Konzentrationen als der Stamm *E. coli* BL21 pBT10.

### Beispiel 6: Oxidation von iso-Butan durch E. coli mit der Monooxygenase (alkBGT) aus P. putida GPO1

Der Arbeitsablauf erfolgt analog dem Beispiel 1. Verwendet wird nur der Stamm *E. coli* W3110 pBT10. Der Konversionspuffer besteht aus 70 mM Na⁺/K⁺-phosphatpuffer, pH7, eingestellt mit 5 % NH₄OH, mit 6,79 g Na₂PO₄, 3 g KH₂PO₄, 0,5 g NaCl, 0,49 g MgSO₄*7H₂O, 15 ml TE und 50 µg Kanamycin pro Liter.

Die Begasung erfolgt wie in Beispiel 1 nur mit einer Mischung aus 25 % i-Butan und 75 % synthetischer Luft.

### Ergebnisse

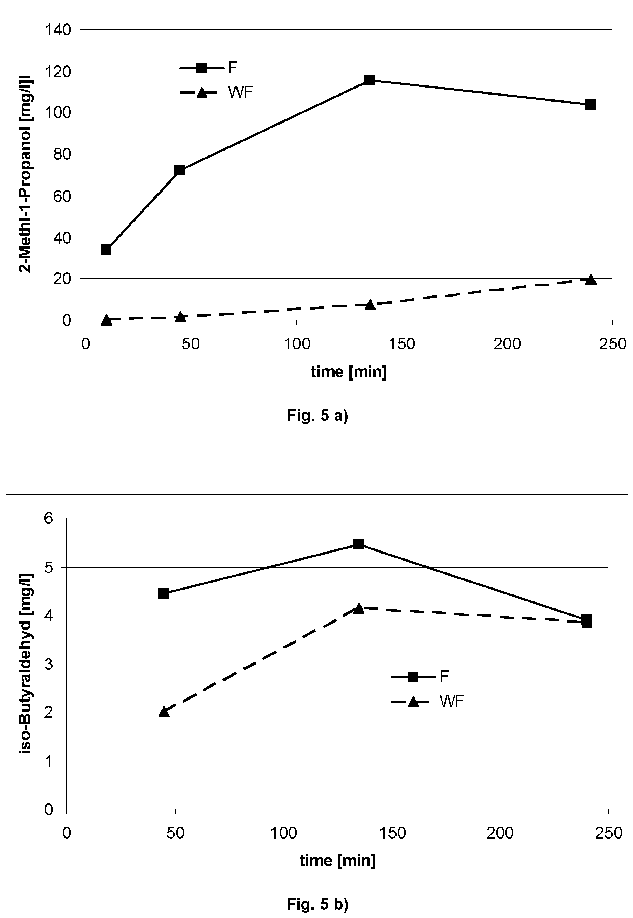

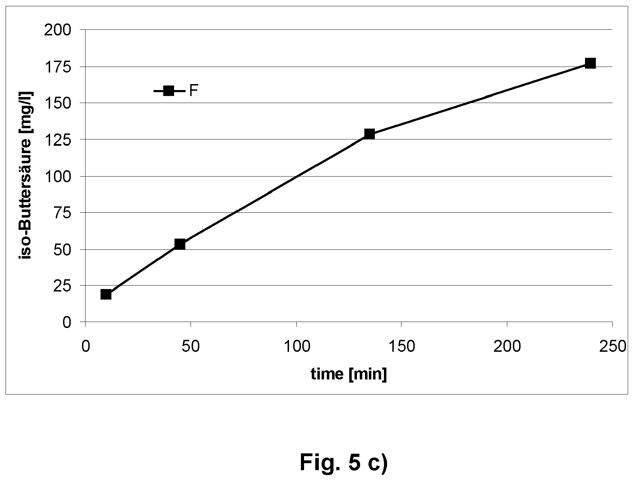

Die Ergebnisse sind in den Figuren **5 a)** **- c)** dargestellt. An Oxidationsprodukten von i-Butan werden i-Butanol, i-Buttersäure, tert.-Butanol und i-Butyraldehyd gefunden.

### Beispiel 7: Oxidation von Butan durch E.coli mit dem Monooxygenase-System alkBG aus Alcanivorax borkumensis

Der für die Oxidation verwendete Stamm enthält ein Plasmid mit den genetischen Informationen für die alkBG-Monooxygenase aus *Alcanivorax borkumensis* SK2 (Datenbankcode CAL18155.1 und CAL18156.1). Die genetischen Informationen für alkST, alkL, sowie die Promotoren für alkS und alkB stammen aus *Pseudomoas putida* GPo1.

### Klonierung des Zielvektors

Für die Vervielfachung wurde der 2x Phusion HF Master Mix von New England Biolabs (NEB, M0531S) nach Herstellerangaben verwendet. Die Vektoren und PCR-Produkte wurden abhängig vom Reinheitsgrad direkt säulengereinigt (*QiaQuick PCR Purification Kit,* Qiagen, Hilden) oder über ein Agarosegel gereinigt und extrahiert (*QiaQuick Gel Extraction Kit,* Qiagen, Hilden). Die Durchführung der PCR, der Agarose-Gel-Elektrophorese, Ethidiumbromidfärbung der DNA und Bestimmung der PCR-Fragmentgrößen erfolgte in der für den Fachmann bekannten Art und Weise. In beiden Fällen konnten PCR-Fragmente der erwarteten Größe bereitgestellt werden. Für die PCR kamen die Primer mit den in SEQ ID NO 1, 2, 3, und 4 angegebenen Sequenzen zum Einsatz.

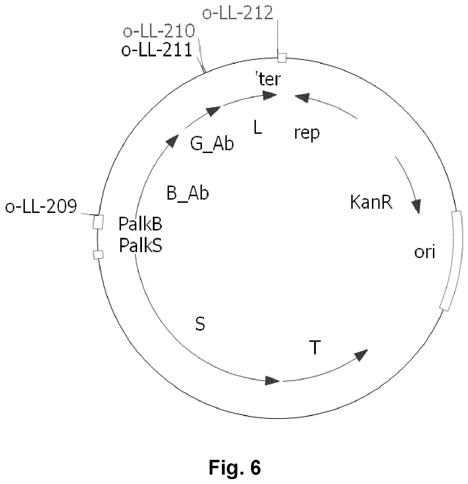

Die aufgereinigten PCR-Produkte wurden in den mit EcoRI-HF + *Ac*/I geschnittenen Vektor pBT10_alkL nach Gelreinigung mittels Rekombination unter Verwendung des In-Fusion HD Cloning Kit nach Anleitung des Herstellers (Clontech Laboratories Inc., Mountain View, CA, USA) kloniert. Die Transformation chemisch kompetenter *E.coli* DH10⊐ (New England Biolabs, Frankfurt) erfolgte nach der für den Fachmann bekannten Art und Weise. Die korrekte Insertion der Zielsequenzen wurde durch Restriktionsanalyse überprüft und die Authentizität der eingebrachten Sequenzen durch DNA-Sequenzierung bestätigt. Der entstandene Vektor wurde mit p-LL-30 (**Fig.** 6 bezeichnet. Die Sequenz des Vektors ist im Sequenzprotokoll unter SEQ ID NO 5 angegeben.

### Spenderorganismen und gespendete Gene:

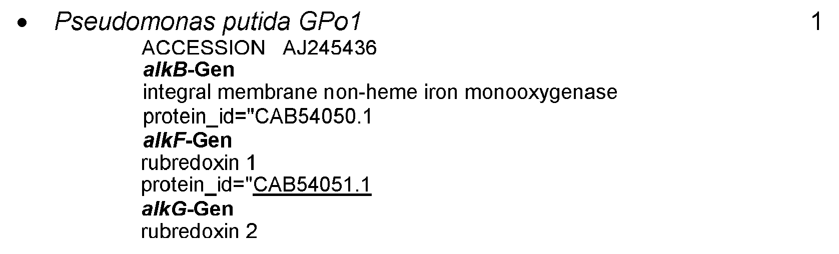

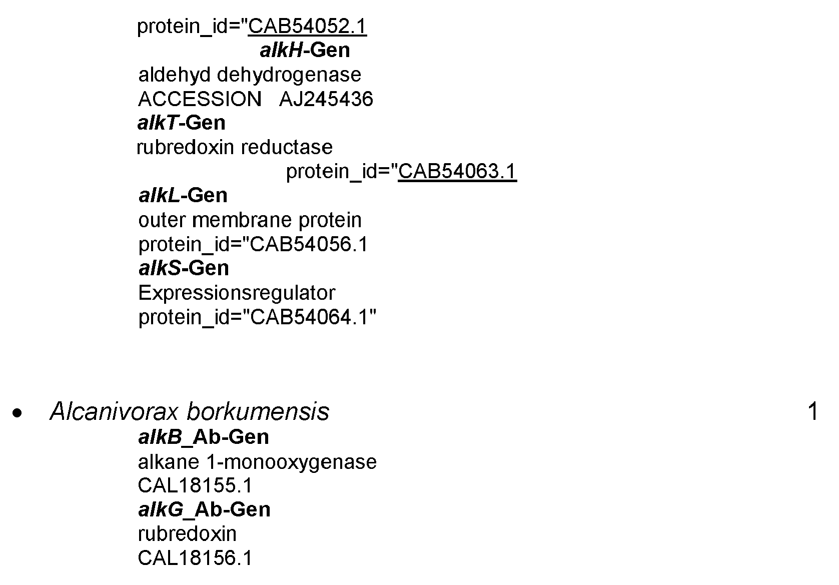

Der Zielvektor wurde in der für den Fachmann bekannten Art und Weise in E. coli W3110 kloniert. Der entstandene Stamm wurde mit E. coli W3110 AN-S-LL-16 bezeichnet.

### Zellanzucht und Biotransformation:

100 µl einer Glycerin-Kryokultur von *E. coli* W3110 EN-S-LL-16 werden auf einer LB-Agarplatte mit 50 µl Kanamycin ausplattiert und für 24 h bei 37°C inkubiert. Die LB-Platten werden aus 1 Liter einer Lösung von Hefeextrakt 5 g, Pepton 10 g, NaCl 0,5 g, Agar-Agar 15 g und Kanamycin 50 µg hergestellt.

Von diesen Platten werden 3 x 25 ml LB-Broth (obige Lösung ohne Agar-Agar) mit 50 µl Kanamycin in einem 100 ml-Schüttelkolben mit Schikanen mit einer Einzelkolonie von der Platte angeimpft. Die Kulturen werden für 24 h bei 37 °C und 200 Upm (Amplitude 2,5 cm) inkubiert.

Danach werden je 25 ml der Kulturbrühe in 175 ml modifiziertes M9-Medium mit folgender Zusammensetzung pro Liter: 15 g Glucose, 6,79 g Na₂PO₄, 3 g KH₂PO₄, 0,5 g NaCl, 2 g NH₄Cl, 15 g Hefeextrakt, 0,49 g MgSO₄*7H₂O, 1 ml TE und 50 µg Kanamycin in 1000 ml Schüttelkolben überimpft. Die Spurenelementlösung (TE) wird pro Liter wie folgt angesetzt: 36,5 g HCl 37 %, 1,91 g MnCl₂*4H₂O, 1,87 g ZnSO₄*7H₂O, 0,84 g Na-EDTA*2H₂O, 0,3 g H₃BO₃, 0,25 g Na₂MoO₄*2H₂O, 4,7 g CaCl₂*2H₂O, 17,3 g FeSO₄*7H₂O und 0,15 g CuCl₂*2H₂O. Der pH wird mit 5 % NH₄OH auf 7,4 eingestellt. Des Weiteren werden pro Kolben 3 Tropfen autokaviertem Antischaum (Delamex) und zugegeben.

Die Kolben werden für 2 Stunden bei 37°C und 180 Upm (Amplitude 2,5 cm) inkubiert. Danach wird die Temperatur auf 25 °C reduziert. Nach 0,5 Stunden bei 25 °C erfolgt die Induktion mit 0,4 mM DCPK. Die Kultur wird für weitere 16 Stunden bei 25 °C und 180 Upm geschüttelt.

Die Kulturen werden vereinigt, in 50 ml-Falcon Tubes abgefüllt und bei 10000 g bei 25 °C für 10 Minuten zentrifugiert. Der Überstand wird verworfen. Die Pellets aus 600 ml Kultur werden in 30 ml Konversionspuffer resuspendiert. Der Konversionspuffer besteht aus 70 mM Ammoniumphosphatpuffer, pH 7 mit 8 g (NH₄)H₂PO₄, 0,5 g NaCl, 0,49 g MgSO₄*7H2O, 1 ml TE und 50 µg Kanamycin pro Liter. Die pH-Einstellung erfolgt mit 25%iger Ammoniaklösung.

150 ml Puffer mit ca. 3 Tropfen autokaviertem Antischaum (Delamex) werden in einem 300 ml-Fermenter vorgelegt. Der Fermenter wird mit einem Gasgemisch von 25 % Butan und 75 % synthetischer Luft über einen Sinterglasperlator mit einer Porengröße von 0,2 µm mit einer Flussrate von 6,5 I_{N}/h begast. Der Fermenter wird in einem Wasserbad auf 30 °C temperiert und mit Hilfe eines Magnetrührers mit 900 Upm gerührt. Die Abluft wird durch eine Waschflasche, die mit 150 ml Wasser gefüllt ist, abgeleitet.

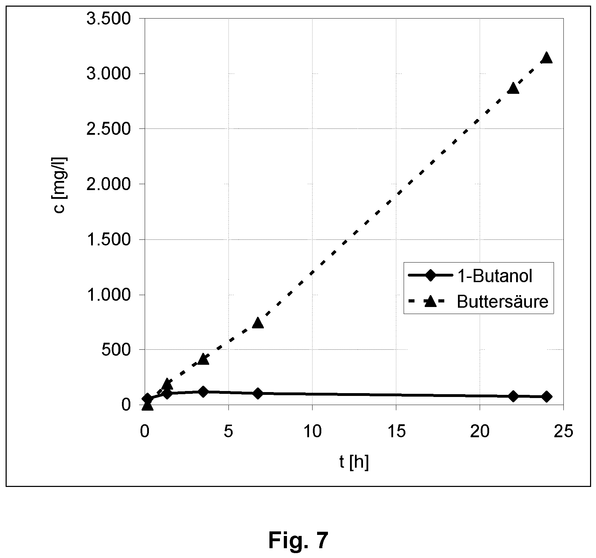

Der Fermenter wird den resuspendierten Vorkultur-Pellets angeimpft. Die OD600 liegt bei ca. 15. Der pH-Wert wird mit 5%iger Ammoniaklösung auf 7,0 geregelt. Die Glucosefeedrate beträgt 1 g/lh. Zu verschiedenen Zeitpunkten werden aus dem Fermenter und der Waschflasche jeweils 5 ml Probe gezogen. Die Fermenterproben werden 10 Minuten bei 10000 g und Raumtemperatur zentrifugiert und der Überstand über einen 0,2 µm-Spritzenvorsatzfilter filtriert. Die Proben werden für die Analytik in HPLC Vials abgefüllt. Die Analytik erfolgt chromatographisch mit HPLC-RID an einer Agilent Technologies 1200-Anlage. Es wird eine Aminex HPX-87H-Säule (300mmx 7,8mm) verwendet. Die Anlage wird mit 10 mM H₂SO₄ als Laufmittel bei einer Flussrate von 0,6 ml/min und einer Säulentemperatur von 40°C betrieben. Standards aller zu analysierender Stoffe werden in Reinstwasser vorbereitet und unter identischen Bedingungen gemessen. Die Auswertung erfolgt über Vergleich der Retentionszeiten. Zusätzlich wird zu jedem Probenahmezeitpunkt eine 2 ml-Probe zu Bestimmung von pH, OD und Glucosekonzentration aus dem Fermenter gezogen. Der pH wird an einem externen pH-Meter gemessen, die OD spektrometrisch bei 600 nm und der Glucosegehalt mit einem biochemischen Analysator (YSI Select 2700 Von Kreienbaum) bestimmt. Die Ergebnisse sind in **Fig. 7** zusammengefasst.

### Literaturstellen:

A. Cornish-Bowden (1995), Fundamentals of Enzym Kinetics, Portland Press Limited, 1995
DE 60216245 (2007): Functional Display of Polypeptides
Sambrook/Fritsch/Maniatis (1989): Molecular Cloning: A Laboratory Manual, Cold Spring Harbor Laboratory Press, 2nd edition
Fuchs/Schlegel (2007) Allgemeine Mikrobiologie, 2008, Georg Thieme Verlag
EP 98137 (1984) A microbiological process for the oxidation of alkanes, vinyl compounds and secondary alcohols
WO 2009/077461: ω-Aminocarbonsäuren oder ihre Lactame, herstellende, rekombinante Zellen
A. Lesk (2008), Introduction to bioinformatics, 3rd edition
F. M. Ausubel (1995), Current Protocols in Molecular Biology. John Wiley & Sons, Inc.
C Grant, J M Woodley, F Baganz (2011): Enzyme and Microbial Technology 48, 480-486

## Patentansprüche

1. Verwendung einer Oxidoreduktase vom AlkB-Typ zur Herstellung in Anwesenheit von Sauerstoff eines Gemisches von Oxidationsprodukten eines Alkans, wobei das Verhältnis von Carbonsäure zu Alkohol unter den Oxidationsprodukten größer als 1:1 ist, wobei es sich bei dem Alkan um ein gasförmiges Alkan mit 3 oder 4 Kohlenstoffatomen, bevorzugt um Butan, handelt,
wobei es sich bei der Oxidoreduktase vom AlkB-Typ um AlkB aus *Pseudomonas putida* GPO1 oder eine Aminosäuresequenz, die mit Hinblick auf die Aminosäuresequenz von AlkB aus *Pseudomonas putida* GPO1 eine Identität von 70 % oder mehr Prozent aufweist und die gleiche Funktion wie die ursprüngliche AlkB aufweist, handelt.

2. Verwendung nach Anspruch 1, wobei es sich bei dem Alkan um ein verzweigtes Alkan, bevorzugt mit vier Kohlenstoffatomen, noch bevorzugter Isobutan, handelt.

3. Verwendung nach einem der Ansprüche 1 bis 2, wobei die Oxidoreduktase vom AlkB-Typ in Form von einem Ganzzellkatalysator bereitgestellt wird

4. Verwendung nach einem der Ansprüche 1 bis 2, wobei die Oxidoreduktase vom AlkB-Typ in Form von einem aufgereinigten Polypeptid bereitgestellt wird.

5. Verwendung nach einem der Ansprüche 1 bis 3, wobei das Verhältnis von Carbonsäure zu Alkohol unter den Oxidationsprodukten größer als 5:1, bevorzugt größer als 12:1 ist, noch bevorzugter größer als 20:1, am bevorzugtesten größer als 40:1 ist.

## Claims

1. Use of a type AlkB oxidoreductase in the presence of oxygen for preparing a mixture of oxidation products of an alkane, wherein the ratio of carboxylic acid to alcohol in the oxidation products is greater than 1:1, wherein the alkane is a gaseous alkane having 3 or 4 carbon atoms, is preferably butane, wherein the type AlkB oxidoreductase is AlkB from *Pseudomonas putida* GPO1 or an amino acid sequence which, with respect to the amino acid sequence of AlkB from *Pseudomonas putida* GPO1, has an identity of 70% or more and has the same function as the original AlkB.

2. Use according to Claim 1, wherein the alkane is a branched alkane, preferably having 4 carbon atoms, more preferably isobutane.

3. Use according to either of Claims 1 and 2, wherein the type AlkB oxidoreductase is provided in the form of a whole-cell catalyst.

4. Use according to either of Claims 1 and 2, wherein the type AlkB oxidoreductase is provided in the form of a purified polypeptide.

5. Use according to any of Claims 1 to 3, wherein the ratio of carboxylic acid to alcohol in the oxidation products is greater than 5:1, preferably greater than 12:1, more preferably greater than 20:1, most preferably greater than 40:1.

## Revendications

1. Utilisation d'une oxydoréductase de type AlkB pour la fabrication en présence d'oxygène d'un mélange de produits d'oxydation d'un alcane, dans laquelle le rapport entre l'acide carboxylique et l'alcool parmi les produits d'oxydation est supérieur à 1:1, l'alcane étant un alcane gazeux contenant 3 ou 4 atomes de carbone, de préférence le butane,
l'oxydoréductase de type AlkB étant l'AlkB de *Pseudomonas putida* GPO1 ou une séquence d'acides aminés qui présente une identité de 70 % ou plus par rapport à la séquence d'acides aminés d'AlkB de *Pseudomonas putida* GPO1 et présente la même fonction que l'AlkB originel.

2. Utilisation selon la revendication 1, dans laquelle l'alcane est un alcane ramifié, de préférence contenant quatre atomes de carbone, de manière davantage préférée l'isobutane.

3. Utilisation selon l'une quelconque des revendications 1 à 2, dans laquelle l'oxydoréductase de type AlkB est préparée sous la forme d'un catalyseur à cellules entières.

4. Utilisation selon l'une quelconque des revendications 1 à 2, dans laquelle l'oxydoréductase de type AlkB est préparée sous la forme d'un polypeptide purifié.

5. Utilisation selon l'une quelconque des revendications 1 à 3, dans laquelle le rapport entre l'acide carboxylique et l'alcool parmi les produits d'oxydation est supérieur à 5:1, de préférence supérieur à 12:1, de manière davantage préférée supérieur à 20:1, de manière préférée entre toutes supérieur à 40:1.


### SEQUENCE LISTING

<110> Evonik Industries AG
<120> Biological alkane oxidation
<130> 201100451
<160> 5
<170> PatentIn version 3.5
<210> 1
   <211> 39
   <212> DNA
   <213> artificial
<220>
   <223> Primer
<400> 1
   aaaaattgga gaattatgtc agagaacatt ttaaccgag 39
<210> 2
   <211> 24
   <212> DNA
   <213> Primer
<400> 2
   ctaatcttca tggaggacat agtc 24
<210> 3
   <211> 39
   <212> DNA
   <213> artificial
<220>
   <223> primer
<400> 3
   ctccatgaag attagtcgac ctgtaacgac aacaaaacg 39
<210> 4
   <211> 39
   <212> DNA
   <213> artificial
<220>
   <223> primer
<400> 4
   cgttgcttcg caacgttaga aaacatatga cgcaccaag 39
<210> 5
   <211> 11353
   <212> DNA
   <213> artificial
<220>
   <223> vector
<400> 5
   gcccggaggg tggcgggcag gacgcccgcc ataaactgcc aggcatcaaa ttaagcagaa 60
   ggccatcctg acggatggcc tttttgcgtt tctacaaact cttttgttta tttttctaaa 120
   tacattcaaa tatgtatccg ctcatgagac aataaccctg ataaatgctt caataatgca 180
   gcctgaaagg caggccgggc cgtggtggcc acggcctcta ggccagatcc agcggcatct 240
   gggttagtcg agcgcgggcc gcttcccatg tctcaccagg gcgagcctgt ttcgcgatct 300
   cagcatctga aatcttcccg gccttgcgct tcgctggggc cttacccacc gccttggcgg 360
   gcttcttcgg tccaaaactg aacaacagat gtgtgacctt gcgcccggtc tttcgctgcg 420
   cccactccac ctgtagcggg ctgtgctcgt tgatctgcgt cacggctgga tcaagcactc 480
   gcaacttgaa gtccttgatc gagggatacc ggccttccag ttgaaaccac tttcgcagct 540
   ggtcaatttc tatttcgcgc tggccgatgc tgtcccattg catgagcagc tcgtaaagcc 600
   tgatcgcgtg ggtgctgtcc atcttggcca cgtcagccaa ggcgtatttg gtgaactgtt 660
   tggtgagttc cgtcaggtac ggcagcatgt ctttggtgaa cctgagttct acacggccct 720
   caccctcccg gtagatgatt gtttgcaccc agccggtaat catcacactc ggtcttttcc 780
   ccttgccatt gggctcttgg gttaaccgga cttcccgccg tttcaggcgc agggccgctt 840
   ctttgagctg gttgtaggaa gattcgatag ggacacccgc catcgtcgct atgtcctccg 900
   ccgtcactga atacatcact tcatcggtga caggctcgct cctcttcacc tggctaatac 960
   aggccagaac gatccgctgt tcctgaacac tgaggcgata cgcggcctcg accagggcat 1020
   tgcttttgta aaccattggg ggtgaggcca cgttcgacat tccttgtgta taaggggaca 1080
   ctgtatctgc gtcccacaat acaacaaatc cgtcccttta caacaacaaa tccgtccctt 1140
   cttaacaaca aatccgtccc ttaatggcaa caaatccgtc cctttttaaa ctctacaggc 1200
   cacggattac gtggcctgta gacgtcctaa aaggtttaaa agggaaaagg aagaaaaggg 1260
   tggaaacgca aaaaacgcac cactacgtgg ccccgttggg gccgcatttg tgcccctgaa 1320
   ggggcggggg aggcgtctgg gcaatccccg ttttaccagt cccctatcgc cgcctgagag 1380
   ggcgcaggaa gcgagtaatc agggtatcga ggcggattca cccttggcgt ccaaccagcg 1440
   gcaccagcgg cgcctgagag gcgaattgac ataagcctgt tcggttcgta aactgtaatg 1500
   caagtagcgt atgcgctcac gcaactggtc cagaaccttg accgaacgca gcggtggtaa 1560
   cggcgcagtg gcggttttca tggcttgtta tgactgtttt tttgtacagt ctatgcctcg 1620
   ggcatccaat cgatgggaag ccctgcaaag taaactggat ggctttcttg ccgccaagga 1680
   tctgatggcg caggggatca agatctgatc aagagacagg atgaggatcg tttcgcatga 1740
   ttgaacaaga tggattgcac gcaggttctc cggccgcttg ggtggagagg ctattcggct 1800
   atgactgggc acaacagaca atcggctgct ctgatgccgc cgtgttccgg ctgtcagcgc 1860
   aggggcgccc ggttcttttt gtcaagaccg acctgtccgg tgccctgaat gaactgcagg 1920
   acgaggcagc gcggctatcg tggctggcca cgacgggcgt tccttgcgca gctgtgctcg 1980
   acgttgtcac tgaagcggga agggactggc tgctattggg cgaagtgccg gggcaggatc 2040
   tcctgtcatc tcaccttgct cctgccgaga aagtatccat catggctgat gcaatgcggc 2100
   ggctgcatac gcttgatccg gctacctgcc cattcgacca ccaagcgaaa catcgcatcg 2160
   agcgagcacg tactcggatg gaagccggtc ttgtcgatca ggatgatctg gacgaagagc 2220
   atcaggggct cgcgccagcc gaactgttcg ccaggctcaa ggcgcgcatg cccgacggcg 2280
   aggatctcgt cgtgacccat ggcgatgcct gcttgccgaa tatcatggtg gaaaatggcc 2340
   gcttttctgg attcatcgac tgtggccggc tgggtgtggc ggaccgctat caggacatag 2400
   cgttggctac ccgtgatatt gctgaagagc ttggcggcga atgggctgac cgcttcctcg 2460
   tgctttacgg tatcgccgct cccgattcgc agcgcatcgc cttctatcgc cttcttgacg 2520
   agttcttctg agcgggactc tggggttcga aatgaccgac caatcgattg gtaactgtca 2580
   gaccaagttt actcatatat actttagatt gatttaaaac ttcattttta atttaaaagg 2640
   atctaggtga agatcctttt tgataatctc atgaccaaaa tcccttaacg tgagttttcg 2700
   ttccactgag cgtcagaccc cgtagaaaag atcaaaggat cttcttgaga tccttttttt 2760
   ctgcgcgtaa tctgctgctt gcaaacaaaa aaaccaccgc taccagcggt ggtttgtttg 2820
   ccggatcaag agctaccaac tctttttccg aaggtaactg gcttcagcag agcgcagata 2880
   ccaaatactg tccttctagt gtagccgtag ttaggccacc acttcaagaa ctctgtagca 2940
   ccgcctacat acctcgctct gctaatcctg ttaccagtgg ctgctgccag tggcgataag 3000
   tcgtgtctta ccgggttgga ctcaagacga tagttaccgg ataaggcgca gcggtcgggc 3060
   tgaacggggg gttcgtgcac acagcccagc ttggagcgaa cgatctacac cgaactgaga 3120
   tacctacagc gtgagctatg agaaagcgcc acgcttcccg aagggagaaa ggcggacagg 3180
   tatccggtaa gcggcagagt cggaacagga gagcgcacga gggagcttcc agggggaaac 3240
   gcctggtatc tttatagtcc tgtcgggttt cgccacctct gacttgagcg tcgatttttg 3300
   tgatgctcgt caggggggcg gagcctatgg aaaaacgcca gcaacgcggc ctttttacgg 3360
   ttcctggcct tttgctggcc ttttgctcac atgttctttc ctgcgttatc ccctgattct 3420
   gtggataacc gtattaccgc ctttgagtga gctgataccg ctcgccgcag ccgaacgacc 3480
   gagcgcagcg agtcagtgag cgaggaagcg gaagagcgcc tgatgcggta ttttctcctt 3540
   acgcatctgt gcggtatttc acaccgcata ggggatctcc aatcgtgcct tggcgcagcg 3600
   acagccctcg gtcccccaga tagccattga tcttctctcg cctgtcccct cagttcagta 3660
   atttcctgca tttgcctgtt tccagtcggt agatattcca caaaacagca gggaagcagc 3720
   gcttttccgc tgcataaccc tgcttcgggg tcattatagc gattttttcg gtatatccat 3780
   cctttttcgc acgatataca ggattttgcc aaagggttcg tgtagacttt ccttggtgta 3840
   tccaacggcg tcagccgggc aggataggtg aagtaggccc acccgcgagc gggtgttcct 3900
   tcttcactgt cccttattcg cacctggcgg tgctcaacgg gaatcctgct ctgcgaggct 3960
   ggccggctac cgccggcgta acagatgagg gcaagcggat ggctgatgaa accaagccaa 4020
   ccaggaaggg cagcccacct atcaaggtgt actgccttcc agacgaacga agagcgattg 4080
   aggaaaaggc ggcggcggcc ggcatgagcc tgtcggccta cctgctggcc gtcggccagg 4140
   gctacaaaat cacgggcgtc gtggactatg agctcgagaa cgcttaccgc caacacagca 4200
   gtgtatttga ataagagctc gaacatgatc ggatctccca tttcagcaag ggaaatccca 4260
   ccatagccac caccgattta tgtggcgtag aaaacggtca tcaccaaata tggcaatact 4320
   tccccgccca agcgccacat aacagcctgg gccacatttt acgcggtagt tccgcacgta 4380
   cggagtgcgg ggggcggcag gtaactgccg tccctatcgc gaccgttggt tcgagtggct 4440
   gagcatcatt gctaatcagg taattttata ctccctgcaa gcgcaccttg acgtttaggc 4500
   aaatttattg tctcagttgc aatcagtcgc tcctgcttgt acgcaaggac ttctagttca 4560
   agagtttcgt tattaattgc aacaacgagt ttatcgtagt cctttagagc accaagtcct 4620
   tgcagcgcca tccctttaag atcagaccag aaccgtggtg gggttggtgc tggtgttgat 4680
   gtgccacaga tgctacttgc gacaatttga gcgtgtgtaa ccgcattatg aattgtctct 4740
   aaacgtacca tcgttcccca aaaaggattt ctagccattg cgcagtcgcc gattgcatat 4800
   atacttgtat ccgatgtaca catctgatca tcgaccacaa caccattact cacttcaagg 4860
   gccgcctcag ttgccagctc tagctctggg atagcaccga ttccaactac aatcagatcc 4920
   gcctgaattt cttctccact ttcaagtacg cattgttcaa catggccatt cctgcccttt 4980
   atagacgtta atttcgcatt cagcttgaac tcaattcctt cagcctccag gcgggctctg 5040
   actaagtttg ctgctgccgg cgtaaccacg cgcgccatta cacgcggggc ggcttctatc 5100
   actgtgaccc tcttccctaa gcccaccgca gctgaggcga cttcaagccc gattactccg 5160
   ccgcccaaca caacaacaga cgcactctcc acaagtttcc tacgtaaatt tttggcgtct 5220
   tccatactgc gtaaatagca gaccccagac agttcagacc cctcgcaggt taacctacgt 5280
   gcgctagcac ctgttgcaag aatcaatttt tcatacgcgt attcttttcc atctttagaa 5340
   gaaactatct tacgccccac gtcgattgat acaatcggtg tatttaacga aatggtaata 5400
   ttgttattcg tataaaaacc ttctggcttt aatggcactg cggattctgc aatctcactt 5460
   gtcagaaaag ccttggatag aggaggccgc tgataaggcg ccacagactc cctgctaaaa 5520
   atcctaattt cccctttata accatattga cgaagccaga acgcagcatt tactccagct 5580
   gtaccagcgc caacaacaac gattgccata attctctctc cggtatactt ttcactatat 5640
   cacttaatgc cgattatttt agataattcc ttgacgctca gcttcaattg ttgcttgcgt 5700
   gcgattcact acattcaagg tggcaaatat tttcctcata tgccacttta tagcatcttc 5760
   ggtgacatgc atatttgttg ctatttgttt gtttgagcac ccctctttta caagcctcaa 5820
   gacagcaatc tgcttccgtg tcaataaagc gtcagcttta ttctctgcgg actttccaat 5880
   ctcaactatt cgcggaagac taaaagcccc aatcgcttga tctaaattaa ctgctgtgaa 5940
   ggcttcacat gaagccggta ttattcgctc aattaaacat acttcatcaa gaactgtttg 6000
   aaagcattga agctgttttg ctatctccac tgcataaaca atgttaagct gagccttttt 6060
   taaatcaccg gcacctgcct gcgctccggc caaacacaat aatccacgga cttccagctg 6120
   gcccgcgtta attttacggg cttgctgaat agccaataac gctctgtgcg cggcactatg 6180
   aaagttccga tctcgggaaa gcactagtga ttgaacaagc agcaggcgtg cttttagggg 6240
   ggctgagtgc tgtccggaga aaatcttatg atcttcaaga gtttttaaat tatttatgcc 6300
   cgttatgcct tgacagacta agcgctgata gatctcaatt tggctcataa cttccaatct 6360
   tggtagattt ttttcaaccg catgcgcctt cgcccactcc aatatctcaa tggagccatt 6420
   taggtcactc cttccaagcc gccaagctga cacagcacgg catacggaaa aaaacacgtc 6480
   tgtcaccccg tgattggaaa tgaactctaa aattttggag agcttttctt ctgaggtgtc 6540
   caagcagcgc aattcataat gtaactcaag ctctagagcg tcaaacattt tcgaagtaaa 6600
   ctcggattcc atcatctgcg cgcgactgtc tgtgcgtgct tgagttataa tctgcctcgc 6660
   ccagcccatt tttccgcttg ctagggcttg ttgaaacctc gcgacataca gccaaccaaa 6720
   agcaaaattt tgttttgcaa atttattcac ggcttgggcc tgagccagca ccttctccaa 6780
   ctctgcaaat ctatactcac tggcaaaaat aaaagccaaa caggttagcg cggccccttt 6840
   tccaactgcg tttgaatccc caaataaact aatccactta ttacagagct cctcactcga 6900
   aagcatttca tctttcgttg ctttacctat tgcaagcaca agctgcagcc attccttttc 6960
   ttgccattta ttttttttat cggattgtga agataggtct ttaattaact tctctgctcg 7020
   cgcgccttgc tgactgaaat acaataccca cgcgtaacta ataagcacta tgggtttttt 7080
   gtgccaggcc tgcttcggca gctctaacag ccactgtctc agcgcatcta tttcgccctg 7140
   acgaaatgac aaatctaaaa ttattctctc agacatgctg actgcccagc gacagtcatt 7200
   cgcccgtagg gatattcgta ttgcatactg gtattcacct ctacgccaat gccagaaagc 7260
   tgcacgctta agcaggtagg atcttttagc aggattttca gtccaagtaa tttctcgtag 7320
   aaaattacgc agtactggat gcagtgtaaa ctgcgctggc tcaccgctca catggcgaag 7380
   caacatgtaa ttagtgctta aatacttaat acatgagacc ccattgacgc atttgaatac 7440
   ataattgtat tgatcaggcg tcacgaaatc gagcaatgaa gaatttgcaa gaaaaacacg 7500
   atagcgctcg ggaatcgcct caaatatttc atccctaaag taattgtcta cttcaactac 7560
   tgctgaaata tgcttggccg gcaactcacg ctttaacaaa aaaactacaa gagcaggcca 7620
   cccctcaact tcttgcacca aggtctctat ctgttcttca ggaactccaa gaacagactc 7680
   tgcctccgct aacgccaccg cctcttctgc gctaaaggcc aagtctttct cggtgtactc 7740
   ccgcatagcg cctgcaagtt taagctgcga gaaccctttt attgtattgc ctgcaactgc 7800
   aaacctgata ttttttggtg tatttaacat aaactccata agtgcgtgca acaacggcaa 7860
   gtctaagtca tgattaatat tatccaaaca aactagcgtt tctatctcgt tattcgaggt 7920
   gctctgccaa agactagatg caaggtctcg caagagcgca ggcttgctca caccctctct 7980
   cacacggctg aattttacca tttcgaaagt ttcaagctgc tcaataatct ctgcgcagat 8040
   atcaaattca ctgtaagaac tggctcttaa agaaagccac actgcaggac gtccggctgt 8100
   tctgtggcgt agccactcga acgcaagagc aacggttttc ccatatccag gtggggctct 8160
   gtaaaggcat actctgggag cggctccatc cgcgatactc aatcttggcc gatatatgca 8220
   actatgaact ttggcactta ctagagtcgt aatttgatcc gctccgacct tagcgaccgg 8280
   gaaatcatta tttattatta ttttcattat gctattctcg cgccagctga ctggaaattt 8340
   tcaccatagg ttacggtgtt aaatattaaa actacactta agtgtagtcg gcatgatcgg 8400
   tggtgcaaaa tatttactag ggaaggtctg aagtaggccg ctatttctgg ccgacttcgg 8460
   ccttcgccga ttttgaagac gggcaccggg tcaaaatcga ccagatagct cgctcatttc 8520
   ggtgctttca gccgtcgcga gtagctcgcg gtacctggca tgcttgcggc cagctcgtgt 8580
   ttttccagca gacgacggag caaaaactac ccgtaggtgt agttggcgca agcgtccgat 8640
   tagctcaggt ttaagatgtc gagagtgaga gtgggcggct taactttctc agttaggcat 8700
   aaaattacgt cttaaatctc gtagcgacta atttaataaa aattggagaa ttatgtcaga 8760
   gaacatttta accgagcccc cacgaagtga tgctgataat gagggttatg tggaccgaaa 8820
   gcgccacttg tggattcttt ctgtactgtg gccagcaaca ccaataattg gcctatatct 8880
   cgtatcccaa acagggtgga gtatatggta cggattagta ttaatcctat ggtacggcct 8940
   agtccctttg atcgacacca tgcttggcga ggattattcc aaccctcctg aatccgttgt 9000
   tcctaagctt gaacaagacc gttactacaa agttttaacg tacttaaccg ttcctattca 9060
   ttatgcagcg ttgattataa gtgcctggtg ggtatctacc caaccaatag gggtattcga 9120
   gtttttagct ctcgcccttt ctttgggcat tgttaatgga ctggcactca acacaggcca 9180
   tgaactcggg cataaaaaag aaacctttga ccgctggatg gccaagcttg tactggccgt 9240
   ggtcggatat ggtcacttct tcattgaaca caataaaggg catcatcgtg acgtagcaac 9300
   accgatggac ccggctacat cccgcatggg cgaatctatt tatacgtttt cactgcgtga 9360
   aattcctggt gcctttaaac gggcatgggg cttggaagag cagcgcctca gccgttgcgg 9420
   caaaagcgta tggagcctag ataatgaagt cttacagcct atgattttga cggtagtgct 9480
   ttatgccgca ttgctggcat ttttcggtcc tttaatgctc atctttttgc ccattcaaat 9540
   ggccttcggc tggtggcagc tgaccagtgc caattatatt gagcactacg gactgctgcg 9600
   tgaaaagctg ccgaacgggc gttacgagca tcaaaaaccc catcattcat ggaattcaaa 9660
   ccatgtaatg tcgaacctca tcctgtttca tctgcaacgt cattcagatc accatgcgca 9720
   tcctacaaga tcttatcaat cacttaggga cttcagcgac cttcccaccc tgcctacggg 9780
   ctaccctggg atgttcttcg tcgcattctt tccctcctgg tttcgttcac taatggatga 9840
   tcgggtgatg gagtgggcgc acggagacat taataagatc cagattcagc cgggaatgcg 9900
   tgaattctat gagcaaaaat ttggagtaaa gggttcggag tcacccgata caaccgttgc 9960
   caaataatcg ccaacagaaa tccatctatt aaagctcgtg ctttcacact ttgaggaaca 10020
   gcgcccggcg tgtacgtcgg gcgccattaa ctaaagacaa taatattggt tcaaaggtgc 10080
   tttaaatggc taaatatcaa tgccccgatt gcgaatatat atacgatgaa gtcgctggcc 10140
   acccacacga aggcttcccc ccaggaacgt cttgggaaac gattcctgaa gagtgggcct 10200
   gcccagactg tgcagtaagg gataaagctg acttcgtagt aatagaatcc ggttccgcgt 10260
   ccccggcgtc tggcgcggcc accccagaag tgcgcactgc taccacccca cctaaggcag 10320
   aggcttcacc tcaaaaatca acgggggcct cgactccttc agctaacaat aaagccaaag 10380
   caaaggctaa agccaaaccc gcacgggcaa aatcgtctaa agactccacc ggcaaagaga 10440
   ccacctttcg taaatggatc tgtatcactt gcggtcacat ttatgatgaa gctcttggcg 10500
   atgaaactga agggttcgcg ccaggcactc tttttgaaga tatcccggac gattggtgct 10560
   gtcccgactg tggtgccaca aaagaggact atgtcctcca tgaagattag tcgacctgta 10620
   acgacaacaa aacgagggta gcacaatgag tttttctaat tataaagtaa tcgcgatgcc 10680
   ggtgttggtt gctaattttg ttttgggggc ggccactgca tgggcgaatg aaaattatcc 10740
   ggcgaaatct gctggctata atcagggtga ctgggtcgct agcttcaatt tttctaaggt 10800
   ctatgtgggt gaggagcttg gcgatctaaa tgttggaggg ggggctttgc caaatgctga 10860
   tgtaagtatt ggtaatgata caacacttac gtttgatatc gcctattttg ttagctcaaa 10920
   tatagcggtg gatttttttg ttggggtgcc agctagggct aaatttcaag gtgagaaatc 10980
   aatctcctcg ctgggaagag tcagtgaagt tgattacggc cctgcaattc tttcgcttca 11040
   atatcattac gatagctttg agcgacttta tccatatgtt ggggttggtg ttggtcgggt 11100
   gctatttttt gataaaaccg acggtgcttt gagttcgttt gatattaagg ataaatgggc 11160
   gcctgctttt caggttggcc ttagatatga ccttggtaac tcatggatgc taaattcaga 11220
   tgtgcgttat attcctttca aaacggacgt cacaggtact cttggcccgg ttcctgtttc 11280
   tactaaaatt gaggttgatc ctttcattct cagtcttggt gcgtcatatg ttttctaacg     11340
   ttgcgaagca acg                                                        11353